Europäisches Patentamt

European Patent Office

Office européen des brevets

(19)

(11) Publication number: **0 334 958**

**A1**

(12) **EUROPEAN PATENT APPLICATION**

published in accordance with Art. 158(3) EPC

(21) Application number: 87907820.2

(22) Date of filing: 26.11.87

Data of the international application taken as a basis:

(86) International application number:
PCT/JP87/00919

(87) International publication number:
WO88/04668 (30.06.88 88/14)

(51) Int. Cl.³: **C 07 K 15/04**
//C12N5/00, C12N15/00,
C12P21/00, A61K39/395,
G01N33/577, (C12P21/00,
C12R1:91)

(30) Priority: 15.12.86 JP 298167/86

(43) Date of publication of application:
04.10.89 Bulletin 89/40

(84) Designated Contracting States:
AT BE CH DE FR GB IT LI LU NL SE

(71) Applicant: NIPPON SHINYAKU CO., LTD.
14, Kisshoinnishinoshomonguchicho
Minami-ku Kyoto-shi Kyoto 601(JP)

(72) Inventor: KAKUNAGA, Takeo
4-11, Midorigaoka 5-chome
Toyonaka-shi Osaka 560(JP)

(74) Representative: Wey, Hans-Heinrich, Dipl.-Ing.
Patentanwälte Wey & Partner Widenmayerstrasse 49
D-8000 München 22(DE)

(54) MONOCLONAL ANTIBODIES.

(57) Monoclonal antibodies capable of specifically recognizing fodrin and having a subclass of $G_2$ ($G_{2a}$, $G_{2b}$). The antibodies can detect fodrin of various origins in addition to animal species and from denatured to native origins. Some of the obtained monoclonal antibodies can detect a third subunit which cannot be detected and polyclonal antibodies. The antibodies can find wide applications to cancer, differentiation of cells, myotonic dystrophy, etc. Particularly with cancer generation, a monoclonal antibody (15A1) capable of specifically recognizing a fodrin β subunit enables diagnosis of the extent of malignance of cancer and differentiation. Identification and determination of fodrin which is a cell-backing protein and which participate in an information transfer system are important for clarifying the mechanism of various diseases, and hence the monoclonal antibodies are quite important from the medical and physiological points of view.

SPECIFICATION

MONOCLONAL ANTIBODIES

## Field of the Invention

The present invention relates to monoclonal antibodies capable of specifically recognizing a fodrin.

Fodrin is a kind of cytoskeleton protein and also called calspectin.    Two subunits having molecular weights of 240 K ($\alpha$) and 235 K ($\beta$) are known.

## Background of the Invention

Cytoplasm constituting a cell looks like a jelly state in which a nucleus and a cell organelle are suspended, but in the inside thereof, 3 kinds of cytoskeletons composed of protein, namely, microfilaments, microtubules and intermediate diameter fibers.  These cytoskeletons not only play a role of maintaining the shape of cells but also participate in cell division or motion and further transfer of cell organelles or vesicles.

In recent years, it has been clarified that cytoskeletons and regulation system thereof are deeply associated with an intracellular information transfer system connecting cell surface receptors with the nucleus and expression of cell functions. Fodrin is present in the synapse site of nerve cells in large quantities so that it is assumed that a fodrin would take an important role in a memory transfer.

On the other hand, where normal cells change to cancer in vitro, morphosis, growth, motion, metabolism, etc. of cells greatly change. In particular, change in morphosis of cells is used as the most characteristic index of cancer generation or transformation from the cells.

It is known that this change in morphosis is well in compliance with change in morphosis of actin cables which are a flux of microfilaments as one of the cytoskeleton. Actin is one of a protein constituting the cytoskeleton and one of the protein connecting actin with cell membrane is a fodrin.

According to recent investigations, it has been made clear that hormones or growth factors bind with receptors present on cell membranes from the outside of cells and participate in phospholipids in the cells or cell membranes or participate in an increase of protein-associated kinase or intracellular Ca ion concentration, resulting in its activation.

Fodrin is a cytoskeleton protein constituting a lining protein of cell membrane and at the same time, it is assumed to directly participate in an information transfer function of cells. Therefore, it has recently come to propose that cell membrane-associated protein having these functions be named membrane skeleton (membrane protein), not cytoskeleton protein hitherto used. It is expected that the results of investigations in this field would find a new importance on cell biology of a fodrin.

Further according to the other finding, it has been made clear that some of the products from carcinogenetic genes produce receptors for growth factors of cell membranes or protein kinase. Therefore, it can be said that it has been clarified that cytoskeleton protein would participate in carcinogenetic mechanism somehow.

According to the aforesaid investigations on transformation in vitro, it has become clear that the quantity of a fodrin in cells decreases by transformation. It has also becoming clear that the fodrin changes its morphosis upon transformation.

From the foregoing, it is suggested that correlationship would exist between the presence of a fodrin and transformation. It is also suggested that identification or quantitative determination of the fodrin in cell would be applicable to a possibility of diagnosis of cancer.

It has also been assumed that a fodrin would also participate in cell differentiation as one of the membrane skeleton.

As a background where cells cause a dynamic change in morphosis thereof, it has been considered that an signal transduction system would take a part in cell membrane. Among them, change in morphosis accompanied by differentiation is associated with qualitative and quantitative change in an amount of the cell membrane synthesized, phosphorylation, etc. In order to learn the actual condition for cell differentiation, it has thus been considered to be important to clarify the relationship between differentiation and change in a fodrin.

It is also suggested that a fodrin would be applicable to diagnosis of specific diseases, for example, muscular dystrophy.

It is considered that muscular dystrophy would be caused by activation of a certain protease in vivo, with some morbid change as a start thereby to decompose cytoskeleton proteins. In a muscular dystrophy model, it is known that a fodrin is decomposed (Proc. Natl. Acad. Sci., 81, 776-780 (1984)), suggesting that trace of the fodrin as a marker would enable to application to diagnosis of this disease.

As has been described in detail, a fodrin is an extremely useful substance for clarification of cell differentiation, clarification of mechanism of transformation, a marker for specific diseases, etc. Enablement to its identification and quantitative determination would result in an extremely revolutionary significance in medical and biochemical fields.

Now the presence of an antibody capable of specifically recognizing enables to identification and quantitative determination by labelling the antibody, which would become a promising means for clarification of cytobiological importance of a fodrin.

The presence of a polyclonal antibody capable of recognizing a fodrin was known. Clarification of qualitative change (decrease) in a fodrin accompanied by transformation is investigations which were enabled by identification and quantitative determination of a fodrin by applying the polyclonal antibody thereto (Koji Owada, "Cell Engineering", vol. 5, No. 4, 314 (1986)).

However, it is known that a fodrin has two subunits of α and β and has a plurality of sites capable of binding many cytoskeleton proteins. Accordingly, the polyclonal antibody recognizes not only a definite site but also indefinite sites, of the fodrin so that any specific effect based on recognition of its specific site could not be expected. Here, if antibodies incapable of recognizing antigens other than a fodrin, namely, monoclonal antibodies capable of specifically recognizing a fodrin were acquired, the aforesaid problem would be solved.

Glenney et al. acquired monoclonal antibodies capable of specifically recognizing a fodrin using their own means (J. Mol. Biol., 167, 275-295 (1983)). Burridge et al. also clarified the presence of monoclonal antibodies capable of specifically recognizing a fodrin (J. Cell Biol., 98, 1363-1377 (1984)).

According to these investigations, monoclonal antibodies acquired by them were antibodies having proteins called IgM and $IgG_1$ as subclass. A monoclonal antibody includes various kinds depending upon method for collection, origin, etc. and each kind has its characteristics. For example, monoclonal antibodies having subclass IgM and subclass $IgG_1$ are inferior in binding capability to protein A later described and fail to apply thereto radioimmunoprecipitation (RIP) as an effective means for quantitative determination of a fodrin.

In light of the publications supra, Glenney et al. utilized, among methods for identification of a fodrin, the Western blotting method, ELISA and a means of immunocytochemistry; however, these methods are not means for recognizing the fodrin in a native state but all for recognizing a fodrin denatured by solubilization, etc. Burridge et al. utilized the Western blotting method and the means of immunocytochemistry but these methods are not those of recognizing a fodrin in a native state.

### Disclosure of the Invention

As described above in detail, identification and quantitative determination of a fodrin are of great significance from the medical and physiological points of view. In addition, it has been desired for its identification and quantitative determination to acquire monoclonal antibodies capable of specifically recognizing a fodrin in a native state.

An object of the present invention is to collect such ideal monoclonal antibodies.

In view of the actual situation described above, the present inventor has devoted himself entirely to the finding of monoclonal antibodies suited for the object and made investigations and as a result, has fortunately succeeded to acquire cells capable of producing the monoclonal antibodies and succeeded to collect monoclonal antibodies capable of recognizing a fodrin from these cells.

The gist of the present invention lies in creating monoclonal antibodies, which subclass is $G_2$ (including $G_2$a and $G_2$b), among those capable of specifically recognizing a fodrin. The monoclonal antibodies acquired by the present inventor are capable of not only reacting with a denatured fodrin in high sensitivity even under severe conditions but also recognizing a fodrin in a native state, as will be later described in the examples.

In addition, the monoclonal antibodies are capable of reacting with a fodrin beyond a scope of species such as swine, human, mouse, etc. and their application can spread over a wide range.

Furthermore, it is also noted that the monoclonal antibodies acquired by the present inventor include those capable of recognizing new subunits (other than $\alpha$ and $\beta$) of a fodrin that could not be recognized with polyclonal antibodies. As will be described in detail in the examples, this is a fact discovered by the present inventor for the first time.

Hereafter, a method for collecting the substances of the present invention will be described in order.

Establishment of a cell line capable of producing the monoclonal antibodies capable of specifically recognizing a fodrin in the present invention is performed by imparting a growth function to cells withdrawn from immunized animal or cells obtained by immunizing cells collected from animal including human in vitro (immunized cells) through fusion of myeloma cells thereto, etc. and repeating selection of the objective cell.

Upon preparation of immunized cells, an antigen (in the present invention, a fodrin becomes the antigen) is prepared prior thereto.

As the antigen, a non-purified fodrin may also be used but desired results are given by using an antigen thoroughly purified.

Upon purification of the antigen, human or animal tissue is homogenized in a conventional manner and then centrifuged, etc. to give precipitates. The precipitates are agitated in a buffer in a conventional manner. The fodrin is present as a lining structure of cell membrane and hence, it is necessary to enhance a salt concentration in the buffer with, for example, potassium chloride or the like. After treating in such a manner, centrifugation is again performed followed by a treatment of precipitation with ammonium sulfate in a conventional manner.

The precipitates are taken out and ammonium sulfate is removed by dialysis to purify. Upon purification, an anionic ion exchange resin column may be used. It is desired to use, for example, Mono Q (registered trademark). Further in gel filtration, various method may be suitably adopted but it is particularly good to use Super Rose Column 6 or Super Rose Column 12 (registered trademark).

Next, as an example of producing the immunized cells, mention may be made of a method for producing the immunized cells which comprises administering an antigen in the body of animal and collecting the animal cells.

As the animal, there may be used animal such as mouse, etc. hitherto provided for experiment in a conventional manner. It is desired that the antigen be administered intraperitoneally, etc. It is appropriate that the administration be effected by mixing the antigen with Freund's complete adjuvant in a conventional manner and administering the mixture. In the present invention, the administration should be repeated several times in several week intervals after the administration. It is sufficient that the administration interval be two weeks and the frequency of the administration is preferably 2 to 4 times.

Then, the animal is sacrificed and the organ such as spleen or the like is ectomized to collect cells in a conventional manner.

Next, cell fusion with myeloma cells for purposes of imparting a growth function to the immunized cells will be described below.

Myeloma cells used herein are those obtained by culturing, for example, X63-Ag8 653 strain, etc., for example, in medium containing FCS (bovine fetal serum). Cells at the logarithmic growth phase are desirably used.

As the method for cell fusion, there may be used a method which comprises mixing immunized cells and myeloma cells in a cell count ratio ranging from 1 : 1 to 10 : 1 and then using a fusing agent such as polyethylene glycol or electric stimulation, etc.

The cells after fusion are cultured in so called HAT medium supplemented with hypoxanthine, aminoputerine and thymidine, immediately or after preincubation in ordinary medium, whereby only cells fused in combination of immunized cells and myeloma cells can be selected.

In the present invention, selection of antibody-producing cells can be performed while confirming an antibody titer by enzyme-linked immunoadsorbent assay (ELISA), etc.

The cells in which an antibody titer could be confirmed can be selected by repeating incubation in a well plate and in a Petri dish in a conventional manner and performing antibody productivity, immunochemical test of produced antibodies, antigen specificity test, etc.

Further the selection may also be performed using a micromanipulator or a cell sorter.

The cells in accordance with the present invention are capable of producing the monoclonal antibodies in accordance with the present invention continuously in an ordinary state. Therefore, where the monoclonal antibodies in accordance with the present invention are utilized, the supernatant of culture solution of the cells in accordance with the present invention can directly be utilized as a solution of the monoclonal antibodies in accordance with the present invention as it is.

Further the monoclonal antibodies in accordance with the present invention can be produced by administering antibody-producing cells to the body of animal ordinarily provided for experiment, e.g., mouse, etc. (for example, intraperitoneally) and collecting the fluid from the animal, for example, ascites, etc.

In order to purify and collect the monoclonal antibodies in accordance with the present invention from the culture solution of the cells in accordance with the present invention or the ascites, the monoclonal antibodies can be collected, for example, by precipitation with ammonium sulfate, ion exchange chromatography, etc.

The thus collected monoclonal antibodies can be stored as stable substance by adding thereto, for example, various buffer solutions and if necessary, a salt or further an azide, etc., or by means of freeze drying, etc.

Identification of each class of immunoglobulins in these antibodies can be made by ELISA using class-specific antibodies.

Using the thus obtained monoclonal antibodies capable of specifically recognizing a fodrin, identification and quantitative determination of the fodrin in a specimen derived from the organism can be made.

The method for identification of a fodrin using the monoclonal antibodies in accordance with the present invention which is characterized by applying the Western blotting method will be described below.

This method is applicable to identification and quantitative determination of fodrin present in a specimen of organism origin. That is, a fodrin or a specimen of organism origin containing a fodrin is fractionated by SDS-polyacrylamide gel electrophoresis in a conventional manner and then blotted to a nylon membrane or a nitrocellulose membrane. The fodrin transferred onto the membrane is bound to the monoclonal antibody, which is then bound to protein A labeled with $^{35}$S or $^{125}$I. In this case, an excess of the monoclonal antibody and protein A are removed every time by thorough washing. By doing so, $^{125}$I or $^{35}$S is present at 240K ($\alpha$) and 235K ($\beta$) which are specific molecular weights of fodrin, or at the location corresponding to the molecular weights of both, in proportion to the amount. Then, $^{125}$I or $^{35}$S can be detected by an RI scanner or an autoradiography thereby to confirm and quantitatively determine the fodrin.

Next, the method for identification of fodrin characterized by applying RIP thereto will be described.

In the RIP, cells which become a sample are cultured in a culture liquid containing, for example, methione labeled with $^{35}$S or the like so that incorporation into the cells which become a sample is conducted. Thereafter, buffer for solubilization is added to the sample and the lysate is then obtained. The monoclonal antibody in accordance with the present invention is added to the lysate. By mixing them, antigen-antibody response is completed.

Then, protein A-Sepharose or the like is added thereto in a conventional manner. By repeating centrifugation and washing with buffer, impurities other than the fodrin in a conventional manner are removed. Thereafter, electrophoresis is performed in a conventional manner followed by detection of the radioisotope. Where the fodrin is present in the specimen cells, bands appear at 240K ($\alpha$) and 235K ($\beta$) which are specific molecular weights of fodrin or both, in proportion to the monoclonal antibody used.

Next, the method for detection of fodrin in organism cells characterized by applying the means of immunocytochemistry will be described.

In the case of culture cells, the cells are immobilized with an appropriate fixing solution such as formaldehyde-phosphate buffer (PBS) solution, etc. and if necessary, subjected to a treatment with Triton to make a specimen. The monoclonal antibody in accordance with the present invention is added to the specimen to complete an antigen-antibody response. Then, the system is thoroughly washed with phosphate buffered solution (PBS). After reacting with biotin-bound anti-immunoglobulin as a secondary antibody, the reaction mixture is thoroughly washed. Fluorescence-labeled avidin is reacted with the formed fodrin-monoclonal antibody-biotin-bound secondary antibody complex and the fluorescence is observed by a fluorescent microscope thereby to detect the fodrin in the cells.

In the case of pathological tissue, there may be used a specimen obtained by fixing the tissue with formalin or other appropriate fixing solutions to make a slice, or by fixing a frozen slice with formaldehyde, if necessary, followed by a treatment with a surface active agent, etc. By similar operations, immunocytochemistry can be performed.

Next, an experiment showing the relationship between differentiation and the monoclonal antibodies of the present invention will be described.

By applying a means of conventional cell culture, animal cells are cultured in a Petri dish and one day after, cell differentiation inducer such as dibutyl cyclic AMP, etc. is added thereto. Thereafter, the incubation is continued for 7 days, while applying a conventional means such as exchange of medium, etc. and a protein labeled with $^{35}S$, etc. is added thereto. By applying the aforesaid means of RIP, identification and quantitative determination of fodrin are performed.

As will be described in detail in the examples, it is noted that the amounts of α and β out of the subunits of fodrin are increased in this experiment. In addition, the presence of a third subunit which cannot be detected with polyclonal antibodies has made clear for the first time by applying the monoclonal antibodies of the present invention.

Next, as examples for application of the monoclonal antibodies of the present invention, identification and quantitative determination of fodrin in qualitative and quantitative changes in transformed cells will be described.

Using the monoclonal antibody 15Al in accordance with the present invention, distribution of subunit ß of fodrin was traced. Polyclonal antibody recognizes subunits of both α and ß but 15Al recognizes ß alone. As will be described in Example 4 in detail, transformed cells were prepared by 4 methods of (1) kinase type RNA virus, (2) non-kinase type RNA virus, (3) DNA virus and (4) carcinogenetic chemical. Amounts of fodrin in the cell membrane fraction and the supernatant fraction in the respective cells were quantitatively assayed using 15Al.

The present inventor has found that a fodrin bound to the cell membrane is mainly a complex (dimer) of α and ß and a fodrin released in cytoplasm by transformation does not form any complex but is isolated into α and ß.

In an experiment using a polyclonal antibody, a decrease in the amount of fodrin (α and ß) in total was observed in the transformed cells in (1) through (4) described above. By quantitative assay for fodrin with 15Al, it was that free ß increased. It was also noted that a proportion of the free ß amounted to the whole fodrin was different in each of (1) through (4). It can be said that the phenomenon of decreasing the total amount of fodrin due to transformation which has been hitherto recognized is observed and clarified accurately on a principal level. It is suggested that the presence of free subunit would destroy stability of the membrane and cause disturbance of response of the cells to growth factors or other various
information transfer mechanisms. Such complex information
transfer abnormalities could be grasped by tracing β subunit with
15A1 of the present invention.

The transformed cells of (1) to (4) differ in the extent of
malignancy of each cancer, differ in growing of cancer and differ
also in response to anti-cancer agents.

In the transformed cells, a phenomenon that free fodrin is
again reverted to (α + β) type in the cell membrane because of
cell differentiation. This suggests that transformed cells would
be treated by cell differentiation.

Taking the foregoing collectively into account, it is
apparent that the extent of treatment can be determined by
identification and quantitative determination of fodrin.
Likewise, it is apparent that the extent of transformation can be
determined.

Therefore, the monoclonal antibodies in accordance with the
present invention capable of specifically recognizing β subunit
alone are applicable to diagnosis of cancer.

Industrial Applicability

The present invention enables to acquisition of the
monoclonal antibodies capable of specifically recognizing a
fodrin from not only denatured condition but also to native condition
The present invention has also established a method for
identifying a fodrin in biological materials without interference of other proteins, by applying the antibodies to immunological means such as Western blotting, ELISA, RIP, immunocytochemistry, etc. This is not only a means for accurately learning tissue distribution, intracellular distribution and presence state of fodrin but also a promising means for investigating disappearance or phosphorylation of a fodrin or a fodrin-bound protein which is a contact point between change in structure of cytoskeleton protein and information transfer mechanism in cells. The monoclonal antibodies can find wide applications to clarification of fundamental and important problems in the medical and biological fields such as maintenance of morphosis of cells, motion, division, secretion activity, nerve secretion, memory and further cancer, growing senile, etc.

The monoclonal antibodies of the present invention also include those having characteristics that (1) have no specificity to species, (2) are capable of recognizing not only a denatured fodrin but also a native fodrin, (3) are capable of recognizing even a third subunit other than α and β, among subunits of fodrin, and the like. By properly using the monoclonal antibodies of the present invention and known polyclonal antibodies, for example, by determining a rate of α to β, a rate of β to (α + β) which are subunits of fodrin, etc., such can also apply to more detailed clarification of correlationship of a fodrin in vivo with transformation of cells, differentiation, transformed cells, etc.

POOR QUALITY

## Best Mode for practicing the Invention

Hereafter the present invention will be described below in more detail by referring to examples relating to preparation and applications of the present substances but these examples merely show embodiments of the present invention and the present invention is not deemed to be limited thereto.

### Example 1

#### (1) Purification of antigen

To 100 g of the brain of swine was added a 5-fold amount of imidazole buffer followed by homogenization with Polytron (registered trademark). Thereafter, centrifugation was performed with a super-ultra centrifuging machine for an hour at 100,000 G. The supernatant was removed ant the precipitates were taken out and added to Tris buffer containing 0.6 M potassium chloride followed by agitation for 30 minutes.

Then, centrifugation was performed at 80,000 G for 30 minutes. The precipitates were removed and the supernatant was taken out and 0.258 g/ml of ammonium sulfate was added thereto. The mixture was allowed to stand at 4°C for 30 minutes. Then, centrifugation was performed at 200,000 G for 45 minutes. The precipitates were taken out and 30 ml of Tris buffer was added thereto. After dialysis with the same Tris buffer, gel filtration was performed through a CL-4B (registered trademark) column of 44 mmØ x 90 cm. An ammonium sulfate solution was added to 50% saturation followed by centrifugation (200,000 G, an hour). The precipitates were taken out and dissolved in Tris buffer. The solution was dialyzed to Tris buffer and the dialysate was subjected to Mono Q Column 5/5 ion exchange column chromatography. Gel filtration using Super Rose Column 12 gave 30 mg of fodrin as a single protein.

(2) Production of immunized cells

A mixture of 0.1 ml of a solution containing 100 µg of fodrin obtained in (1) and 0.1 ml of Freund's complete adjuvant (manufactured by DIFCO Co., Ltd.) was intraperitoneally administered to BALB/c female mice of 10 week age. Thereafter, the aforesaid mixture of the fodrin solution and incomplete adjuvant (manufactured by DIFCO Co., Ltd.) was intraperitoneally administered twice in 2 week intervals. Then, the mice were sacrificed by cervical dislocation                and the spleen was aseptically collected.

About 1 g of the spleen described above was put on a selector (manufactured by BELCO Co., Ltd.) and passed through a mesh of about 10 µ while supplying Dulbecco-modified MEM (D-MEM) to give spleen cells suspended in D-MEM. The suspension was centrifuged at 1000 G for 5 minutes to collect spleen cells.

The aforesaid cells were put in 1 ml of a solution obtained by adding 20 mM HEPES buffer (pH 7.4) to 0.84% ammonium chloride solution to cause hemolysis. The cells were obtained by centrifugation at 1000 G for 5 minutes. The cells were again suspended in D-MEM medium.

(3)  Production of myeloma cells

8-Azaguanine-resistant and immunoglobulin non-secretion type mouse myeloma cell line X63-Ag8 653 strain was cultured in D-MEM medium supplemented with 20% fetal calf serum (FCS) in an incubator of 10% $CO_2$ at 37°C.  Cells at the logarithmic growth phase were collected and used for the following operation.

Centrifugation was performed at 1000 G for 5 minutes to collect only the cells.  The cells were further suspended in D-MEM medium and the cell count was counted with a hemocyto-meter.      After centrifugation at 1000 G for 5 minutes again, the cells were suspended in D-MEM medium.

(4)  Cell fusion

The D-MEM medium containing $10^8$ to $3 \times 10^8$ of immunized cells obtained in (2) was mixed with the D-MEM medium containing $10^8$ of immunized cells obtained in (3).  After the mixture was homogenized, it was centrifuged at 1000 G for 5 minutes.  The supernatant was removed to give the precipitates.  To the precipitates was dropwise added 1 ml of a solution containing 25% (w/v) polyethylene glycol 1500 (PEG 1500) (manufactured by Boehringer Co., Ltd.) and 37.5 mM HEPES buffer over a minute.  Thereafter, the mixture was slowly diluted with D-MEM medium to make 10 ml as a whole.

D-MEM medium, 10 ml, containing 20% FCS was added to the system followed by centrifugation at 1000 G for 5 minutes.  D-MEM containing 20% FCS was added to the obtained cells to have $10^6$

- 21 - 0334958

cells/ml. The mixture was put on a 24 well culture plate manufactured by Corning Glass Co., Ltd. in a proportion of 1 ml/well.

Incubation was performed in an incubator of 10% $CO_2$ at 37°C for 24 hours. Then, HAT solution was added to remove cells other than fused cells. Incubation was continued for further 2 weeks.

(5) Measurement of antibody titer by ELISA

Each well of a microtiter plate (No. 001-010-2101) manufactured by Dynatech Laboratories Co., Ltd. was coated with 1 µg/ml of the fodrin obtained in (1).

The supernatant, 150 µl, containing the antibody which was the objective for measurement was taken in the well. After allowing to stand at 37°C in an incubator for 2 hours, the system was washed with PBS (phosphate buffer). Peroxidase-bound sheep anti-mouse whole antibodies were added thereto. After allowing to stand at 37°C in an incubator for an hour and washing with PBS, a color developing reagent (ABTS) was added to form a color for 15 minutes. A stopping solution (0.1 M citrate - 0.02% sodium azide) was added to discontinue the reaction. Then, absorbance of each well was measured with a multiscan manufactured by Titertech Co., Ltd. to calculate the antibody titer.

(6) Selection of antibody-producing cells

The cells in the well confirmed by ELISA were spread over soft agar medium in a Petri dish of 60 mmø. Incubation was performed in 10% $CO_2$ in an incubator of 37°C for 2 weeks to form colonies. The formed colonies were taken out and put on a 24 well culture plate. Antibody activity was again confirmed by ELISA. The cells in the wells, antibody activity of which was confirmed, were spread over soft agar medium in a Petri dish of ~60 mm∅. Incubation was performed in 10% $CO_2$ in an incubator of 37°C for 2 weeks to form colonies. The formed colonies were taken out and put on a 24 well culture plate. Antibody activity was again confirmed by ELISA and useful cells were selected.

(7) Collection of antibodies

(1) The antibody-producing cells obtained in (6) always produce the antibodies of the present invention and hence, the supernatant of culture solution obtained by culturing the antibody-producing cells can be directly used as the antibody solution of the present invention.

(2) Ammonium sulfate was added to the culture solution of the antibody-producing cells obtained in (6) to a final concentration of 30%. After centrifugation, the precipitates were taken out and 20 mM of phosphate buffer of pH 7.4 was added thereto. Dialysis was performed using the same phosphate buffer (containing 0.02% sodium azide) to remove ammonium sulfate. The liquid after the dialysis was freeze dried to give white powders.

(3) Pristane, 0.5 ml, was intraperitoneally injected to BALB/c male mice followed by feeding for 2 weeks. The antibody-producing cells obtained in (6) were injected in $10^6$ to $2 \times 10^6$ cells/mouse followed by feeding for 10 days. Using a

- 23 -

0334958

syringe, about 10 ml of the ascites retained in the peritoneal cavity was collected.

Ammonium sulfate was added to the ascites to a final concentration of 30%. Then, 20 mM of phosphate buffer showing pH of 7.4 was added thereto and dialysis was performed using the same phosphate buffer (containing 0.02% sodium azide) to remove ammonium sulfate. The liquid after the dialysis was freeze dried to give white powders.

(8) <u>Identification for each class of immunoglobulin</u>

Identification of each class of immunoglobulin was made by 3 step ELISA using class-specific antibody.

The supernatant, 150 µl, containing an antibody which was the object to be measured, was taken in a well, which was allowed to stand at 37 °C in an incubator for 2 hours. Then, the system was washed with PBS (phosphate buffer). Class-specific antibodies (products manufactured by Biorad Co., Ltd. were used as IgA, IgG1, IgG2a, IgG2b, IgG3 and IgM) were added followed by reacting at 37 °C for 2 hours. Then the system was thoroughly washed with PBS. Peroxidase-bound sheep anti-mouse whole antibodies were added thereto. The mixture was allowed to stand at 37 °C for an hour in an incubator. After washing with PBS, a color developing reagent (ABTS) was added to form a color for 15 minutes, whereby each class was determined. A part of the results is shown in Table 1.

Table 1

| Reference No. | Antibody No. | Cell Line | Subclass |
|:---:|:---:|:---:|:---:|
| 1 | 10 | 3B2 | $G_2a$ |
| 2 | 26 | 5D3 | $G_2b$ |
| 3 | 32 | 7B4 | $G_2b$ |
| 4 | 51 | 9D2 | $G_2a$ |
| 5 | 107 | 12D4 | $G_2b$ |
| 6 | 116 | 15A1 | $G_2a$ |
| 7 | 133 | 17B3 | $G_2a$ |

Example 2    Identification and quantitative determination of fodrin

(1)   Application of Western blotting method

After fodrin obtained in Example 1 and $^{14}$C-labeled molecular weight marker were subjected to SDS-polyacrylamide gel electrophoresis in a conventional manner, protein in the gel was transferred onto a nylon membrane (manufactured by Biorad Co., Ltd.) by applying a voltage of 10 V/cm at 4 °C for 4 hours using Transblot Cell of Biorad Co., Ltd.  Blocking was performing using Tris buffer (solution A) containing 0.5% skimmed milk and 150 mM sodium chloride.

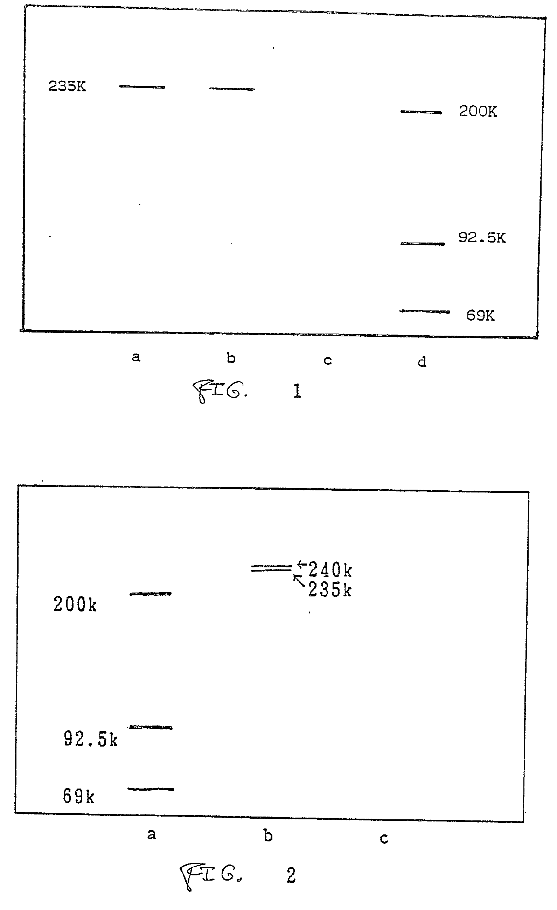

Fodrin monoclonal antibody, which cell line was 15A1, was dissolved in Tris buffer (solution B) containing 0.5% skimmed milk and 150 mM sodium chloride.  After reacting with the membrane described above at room temperature for 2 hours, the reaction product was thoroughly washed with solution B repeatedly. After the fodrin-bound antibody remained on the membrane was reacted with $^{125}$I-protein A (up to $10^7$ dpm) dissolved in solution B, the reaction product was thoroughly washed and dried. An X ray film was exposed to $^{125}$I on the membrane by autoradiography to detect bands of fodrin (Figure 1).

(2) Application of RIP (radioimmunoprecipitation)

Cells used as specimen were cultured in D-MEM medium charged in Petri dish of 100 mm∅. On a day before the system reached a confluent state, $^{35}$S-labeled methionine (Amersham SJ 204) was added in 250 μ Curie/dish followed by incubation in an incubator of 10% $CO_2$ at 37 °C overnight. After the medium was removed, the system was washed with PBS. Then, solubilizing with radioimmunoprecipitation assay (RIPA) buffer, the lysate was adjusted to have a radioactivity of 3 x $10^6$ c.p.m.

Thereto was added the present substance, 24A2. The mixture was slowly agitated at 4 °C for an hour to complete an antigen-antibody response.

To the mixture was added 50 μl of a suspension of protein A-Sepharose CL-4B (manufactured by Pharmacia Co., Ltd.) in RIPA buffer in a concentration of 50% followed by reacting at 4 °C for an hour. After the reaction, centrifugation was performed at 12000 r.p.m. for a minute and the supernatant was discarded.

To the precipitates was added 0.5 ml of RIPA buffer followed by thorough agitation. Centrifugation was performed at 12000 r.p.m. for a minute and the supernatant was discarded. This operation was repeated 5 times.

A sample buffer, 40 μl, for electrophoresis was added followed by heating at 100 °C for 2 minutes.

Thereafter, 5% modified gel electrophoresis (SDS-PAGE) was performed. After protein in the gel was fixed with 7% acetic acid, sensitization was performed with a sensitizer (Amplify manufactured by Amersham Co.).

The gel was dried and an X ray film was exposed thereto to detect bands of protein.

Where fodrin is present, bands can be confirmed at 240K (α) and 235K (ß) or both, depending upon specificity of the antibody used.

A part of examples to see which subunit of the fodrin is recognized using rat cells A-31 will be shown in Table 2.

Table 2

| Reference No. | Antibody No. | Cell Line | Band |
|---|---|---|---|
| 1 | 10 | 3B2 | α |
| 2 | 76 | 11A6 | α |
| 3 | 92 | 12A6 | ß |
| 4 | 110 | 13A5 | α + ß |
| 5 | 135 | 17C5 | α |
| 6 | 133 | 17B3 | α + ß |

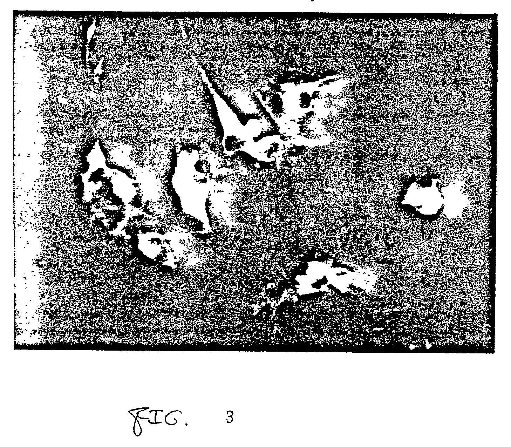

Using human-derived WI-38 cells, runs were carried out in a similar manner, whereby it was confirmed that the precipitation with human fodrin occurred (cf. Table 3 described below and Figure 2).

Table 3

| Reference No. | Antibody No. | Cell Line | RIP |
|---------------|--------------|-----------|-----|
| 1 | 79 | 11B6 | - |
| 2 | 124 | 15C5 | ± |
| 3 | 138 | 18B6 | +++ |
| 4 | 142 | 24A2 | ++ |
| 5 | 145 | 29A5 | + |
| 6 | 133 | 17B3 | α + ß |

(3)  Application to immunocytochemistry

Human neuroblast cultured in Chamber Slide 4804 manufactured by Miles Co., Ltd. was fixed with 3.7% formaldehyde-PBS solution. After washing with PBS 3 times, 10% bovine serum albumin-PBS solution was added to perform blocking for 30 minutes, which was made a sample.

Fodrin monoclonal antibody 15Al diluted with PBS containing 0.02% sodium azide to 200-fold was added to the sample.  After reacting at 4°C overnight, the reaction product was washed with PBS 3 times.

Biotin-mouse whole antibodies (manufactured by Amersham Co., Ltd.) diluted with PBS to 100-fold was reacted as a secondary antibody for an hour followed by repeating washing with PBS 3 times.

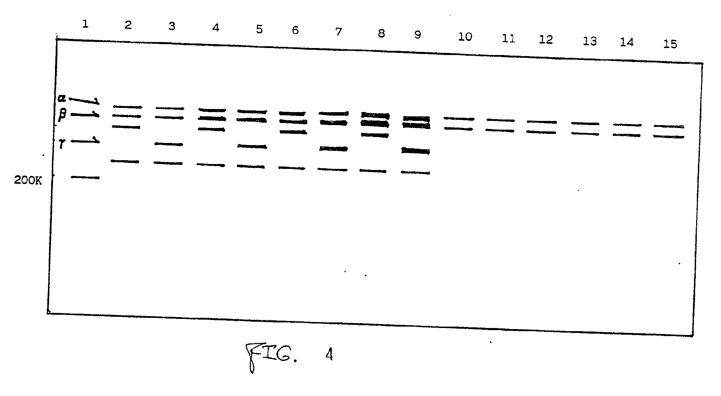

After reacting with a 100-fold diluted streptoavidin-FITC (manufactured by Amersham Co., Ltd.) solution for further an hour at room temperature, the system was thoroughly washed and observed with a fluorescence microscope, whereby fodrin in the neuroblast was confirmed (Figure 3).

Example 3

Identification and quantitative determination of fodrin in cell differentiation

GOTO cells of human neuroblastoma established cell line were cultured in $5 \times 10^4$ cells/ml in a 100 mmØ plastic made-Petri dish. After adherence of the cells was confirmed, 1 mM to 2 mM of dibutyl cyclic AMP or 20 nM of tetradecaphorbol acetate (TPA) was added to the medium to induce differentiation. After incubation for a week in the presence of a differentiation inducer, the cells were labeled with $^{35}$S-methionine or $^{32}$PO$_4$. Then after solubilizing with RIPA buffer, an amount of fodrin synthesized and change in phosphorylation in differentiation induction were detected. The results are shown in Figure 4.

It can be observed that by the differentiation induction, amounts of α and ß subunits synthesized increase but the extent of phosphorylation does not change.

Example 4

Quantitative determination of fodrin in transformed cell

In various in vitro transformation systems, the total amounts of fodrin in cells and amounts of fodrin in the membrane fraction and the supernatant fraction were examined.

Control represents cells prior to transformation. Each transformation method is represented by the following.

(1) RNA virus (kinase type)

(2) RNA virus (non-kinase type)

(3) DNA virus

(4) Carcinogenetic chemical

The total amount of fodrin was quantitatively assayed using known polyclonal antibodies (described in Cell Engineering, 5, No. 4). For quantitative assay for the amounts of fodrin in the membrane fraction and in the supernatant fraction, 15Al of the present invention was used. The membrane fraction and the supernatant fraction were prepared by conventional purification methods. Each sample was separated by electrophoresis and by converting counts on a filter into numeral figures by a γ-scintillation counter by the aforesaid Western blotting method. The results are shown in Table 4.

It is noted that in normal cells as control, a ratio of free β subunit present is approximately 5%, whereas in the cells transformed with RNA virus, in the cells transformed with DNA virus and in the cells transformed with carcinogenetic chemical, the ratio increases by 30 to 50%, 20 to 30% and 10%, respectively.

Table 4

|  | Total Amount | Membrane Fraction | | Supernatant Fraction | |
|---|---|---|---|---|---|
| Control | 100 | 80 | (80%) | 5 | (5%) |
| (1) | 40 | 10 | (25%) | 20 | (50%) |
| (2) | 60 | 20 | (33%) | 20 | (33%) |
| (3) | 60 | 30 | (50%) | 20 | (33%) |
| (4) | 60 | 20 | (33%) | 10 | (17%) |

Each numerical figure represents a relative numerical value when a count of the γ-scintillation counter of the control cells in the total amount is made 100. The numerical value within parenthesis represents a proportion based on the total amount.

Brief Description of the Drawings

Figure 1 indicates results obtained by identification of a fodrin by applying the antibody 15A1 obtained in the examples to the Western blotting method. In the figure, a through d each represents a pattern of purified fodrin, a membrane fraction of rat brain, erythrocyte membrane fraction and a molecular weight marker and numerical figures represent molecular weights.

Figure 2 indicates results examined by applying a fodrin synthesized cultured WI-38 cells by applying a means of RIP. In the figure a, b and c represent a pattern of a molecular weight marker, one obtained when reacting antibody 24A2 with cell lysate and one for control where no antibody is added.

Figure 3 is a photography showing results of the present substance, 15A1, obtained by applying a means of immunocytochemistry to cultured neuroblastoma and represents morphosis of the organism when observed neuroblast with a fluorescence microscope.

Figure 4 represents amounts of a fodrin synthesized and change in phosphorylation when neuroblastoma-established cell line was subjected to differentiation induction, which were practice by means of radioimmunoprecipitation.   Each numeral in the figure indicates the following.

In the figure, 1 is $^{14}$C-labeled molecular weight marker (200K myosin), 2 through 9 each represents results obtained using solubilized lysates labelled with $^{35}$S, wherein polyclonal

anti-fodrin antibody was used in 2, 4, 6 and 8 and 18B6 of the present invention was used in 3, 5, 7 and 9, as the antibody.   2 and 3 indicate control, 4 and 5 are TPA, 6 and 7 are 1 mM dibutyl cyclic AMP and, 8 and 9 are 2 mM dibutyl cyclic AMP.

10 through 15 represent results obtained using solubilized

lysate labeled with $^{35}$P, wherein polyclonal was used in 10, 12 and 14 and 15A1 was used in 11, 13 and 15, as the antibody.   10 and 11 indicate control, 12 and 13 are 20 nM of TPA and, 14 and 15 are 1 mM dibutyl cyclic AMP.

## Claim

Monoclonal antibodies characterized by the following two properties (1) and (2):

(1)  that specifically recognizes a fodrin, and,

(2)  that subclass in each class of immunoglobulin is $G_2$.

235K — 200K

92.5K

69K

a     b     c     d

FIG. 1

←240k
235k

200k

92.5k

69k

a     b     c

FIG. 2

0334958

FIG. 3

FIG. 4

## I. CLASSIFICATION OF SUBJECT MATTER (if several classification symbols apply, indicate all) 3

According to International Patent Classification (IPC) or to both National Classification and IPC

Int.Cl$^4$   C07K15/04 //C12N5/00, 15/00, C12P21/00, A61K39/395,
            G01N33/577, (C12P21/00, C12R1:91)

## II. FIELDS SEARCHED

### Minimum Documentation Searched 4

| Classification System | Classification Symbols |
|---|---|
| IPC | C07K15/04, C12N5/00, 15/00, C12P21/00, A61K39/395 |

Documentation Searched other than Minimum Documentation
to the Extent that such Documents are Included in the Fields Searched 5

## III. DOCUMENTS CONSIDERED TO BE RELEVANT 14

| Category * | Citation of Document, 16 with indication, where appropriate, of the relevant passages 17 | Relevant to Claim No. 18 |
|---|---|---|
| A | Journal of Cell Biology, Vol. 98, No. 4 (1984), Mangeat P., et al [Immuno Precipitation of Nonerythrocyte Spectrin Within Live Cells Following Micro Injection of Specific Antibodies Relation To Cytoskeletal Structures] P1363 - 1377 | 1 |

* Special categories of cited documents: 15

"A"  document defining the general state of the art which is not considered to be of particular relevance

"E"  earlier document but published on or after the international filing date

"L"  document which may throw doubts on priority claim(s) or which is cited· to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance: the claimed invention cannot be considered novel or cannot be considered to involve an inventive step

"Y"  document of·particular relevance; the claimed invention cannot be considered to involve an inventive step when the document is combined with one or more other such documents, such combination being obvious to a person skilled in the art

"&"  document member of the same patent family

## IV. CERTIFICATION

| Date of the Actual Completion of the International Search 2 | Date of Mailing of this International Search Report 2 |
|---|---|
| February 16, 1988 (16.02.88) | February 29, 1988 (29.02.88) |

| International Searching Authority 1 | Signature of Authorized Officer 20 |
|---|---|
| Japanese Patent Office | |

Form PCT/ISA/210 (second sheet) (October 1977)